# EUROPÄISCHE PATENTSCHRIFT

(11) **EP 1 314 392 B1**
(45) Veröffentlichungstag und Bekanntmachung des Hinweises auf die Patenterteilung: **29.10.2003**
(21) Anmeldenummer: 01710058.7
(22) Anmeldetag: 27.11.2001
(51) Int. Cl.: A61B 1/018, A61B 1/00

(54) **Dichtung für ein Endoskop**
Seal for endoscope
Joint d'étanchéité pour endoscope

(43) Veröffentlichungstag der Anmeldung: 28.05.2003
(73) Patentinhaber: Karl Storz GmbH & Co., 78532 Tuttlingen (DE)
(72) Erfinder: Bacher, Uwe, 78532 Tuttlingen (DE); Hermle, Rainer, 78559 Gosheim (DE); Salvermoser, Markus, 78532 Tuttlingen (DE)
(74) Vertreter: Hofmeister, Frank Horst

(56) Entgegenhaltungen:
- EP-A- 0 638 290
- EP-A- 0 696 459
- US-A- 5 779 697
- US-A- 6 117 070

## Beschreibung

Die Erfindung betrifft eine Dichtungsanordnung für ein Endoskop, insbesondere ein Rektoskop, umfassend eine Dichtungsaufnahme des Endoskops, eine Dichtung und ein weiteres Dichtungselement, wobei die Dichtung einen Bund zum Festlegen an der Dichtungsaufnahme des Endoskops, eine mit einer Öffnung zum Durchführen eines medizinischen Instruments versehene Endfläche sowie einen den.Bund und die Endfläche miteinander verbindenden Dichtungsmantel aufweist und die Endfläche relativ zur Dichtungsaufnahme des Endoskops kippbar gelagert ist.

Bei endoskopischen Untersuchungen und/oder endoskopischen Eingriffen werden medizinische Instrumente durch das Endoskop in das Untersuchungsgebiet geführt. Um einerseits das Eindringen von Verunreinigungen in das Endoskop und/oder in den Körper des Patienten zu verhindern und andererseits ein Entweichen des in vielen Anwendungsfällen im Untersuchungsgebiet aufgebrachten Luftoder Gasdrucks zu vermeiden, werden Dichtungen verwendet, die in ihrer Endfläche eine Öffnung zum Durchführen des medizinischen Instruments aufweisen. Starre Dichtungen haben aber den Nachteil, daß das durch die Dichtung hindurchgeführte Instrument aufgrund der Materialsteifigkeit der Dichtung nur schlecht in verschiedene radiale Richtungen verlagerbar ist.

Aus der US-A-5,779,697 ist eine Dichtung für ein Endoskop bekannt, deren mit der Öffnung zum Durchführen eines medizinischen Instruments versehene Endfläche relativ zum Endoskop kippbar gelagert ist. Bei dieser bekannten Dichtung besteht jedoch die Gefahr, daß der Dichtungsmantel beim Herausziehen des medizinischen Instruments aus der Endflächenöffnung überdehnt wird, was schlimmstenfalls zum Abziehen der Dichtung von der Dichtungsaufnahme am Endoskop führen kann.

Davon ausgehend liegt der Erfindung die **Aufgabe** zugrunde, eine Dichtungsanordnung der eingangs genannten Art bereitzustellen, die dem durch die Öffnung der Endfläche geführten medizinische Instrument einen möglichst großen. Bewegungsspielraum bei gleichzeitig sicherem Sitz und hoher Dichtwirkung gewährt.

Die **Lösung** dieser Aufgabenstellung ist erfindungsgemäß dadurch gekennzeichnet, daß die Streckung des Dichtungsmantels in axialer Richtung begrenzbar ist.

Durch diese erfindungsgemäße Ausgestaltung wird verhindert, daß, insbesondere bei der Ausbildung des Dichtungsmantels als Faltenbalg, beim Herausziehen des medizinischen Instruments aus der Öffnung in der Endfläche oder beim Kippen der Dichtung mittels des medizinischen Instruments der Dichtungsmantel in axialer Richtung überdehnt wird.

Diese Begrenzung der axiale Streckung kann dadurch erreicht werden, daß der Bund und die Endfläche über mindestens ein nicht dehnbares Begrenzungselement, insbesondere ein Seil, miteinander verbunden sind. Bei der Verwendung mehrerer Begrenzungselemente sind diese vorteihafterweise gleichmäßig um die Öffnung zur Durchführung des medizinischen Instruments angeordnet.

Gemäß einer ersten praktischen Ausführungsform der Erfindung wird die kippbewegliche Lagerung der Endfläche dadurch erzielt, daß der Dichtungsmantel als mit mindestens einer Falte versehener Faltenbalg ausgebildet ist. Da die Ausbildung der Falten im Dichtungsmantel eine Kippbeweglichkeit der Dichtung bewirkt, wirkt sich das Kippen der Dichtung auch nicht auf die Dichtigkeit der Öffnung in der Endfläche aus, so daß die Bewegung des medizinischen Instruments die Dichtigkeit nicht beeinflußt. Neben der horizontalen Anordnung der mindestens einen Falte ist es auch möglich den Faltenbalg so auszubilden, daß die mindestens eine Falte wendelfömig umlaufend im Material des Dichtungsmantels ausgebildet ist.

Die Kippbeweglichkeit der Dichtung ist durch die Ausbildung mehrer Falten und verschiedenenr Faltenhöhen variierbar. Je nach Anwendungsfall können die Falten eines Faltenbalgs im entspannten Zustand alle die gleiche Höhe aufweisen, oder aber zumindest einzelne Falten des Faltenbalgs im entspannten Zustand eine von den anderen Falten abweichende Höhe aufweisen.

Gemäß einer zweiten erfindungsgemäßen Ausführungsform wird zur Bereitstellung der kippbeweglichen Lagerung der Endfläche vorgeschlagen, daß der Dichtungsmantel mindestens eine Soll-Knickstelle bildend an zumindest einer vorgegebenen Stelle einen Bereich mit einer verringerten Wandstärke aufweist, so daß sich bei einer Kippbewegung des in der Öffnung der Endfläche angeordneten medizinischen Instruments der Bereich mit einer verringerten Wandstärke einbeulen kann, um das Kippen der Endfläche zu gewährleisten.

Die Kippbeweglichkeit der Dichtung ist bei dieser Ausführungsform dadurch variierbar, daß im Material des Dichtungsmantels mehrere Bereiche mit verringerter Wandstärke ausgebildet sind, wobei diese Bereiche mit verringerter Wandstärke in der Höhe und/oder über den Umfang des Dichtungsmantels versetzt zueinander angeordnet sind. Weiterhin wird mit der Erfindung vorgeschlagen, daß neben der Ausbildung einzelner voneinander separierter Bereiche der mindestens eine Bereich mit verringerter Wandstärke als horizontal umlaufender Bereich oder als wendelförmig umlaufender Bereich im Material des Dichtungsmantels ausgebildet ist.

Gemäß einer dritten Ausführungsform der Erfindung erfolgt die kippbewegliche Lagerung der Endfläche der Dichtung indirekt, und zwar dadurch, daß die Dichtungsaufnahme, an der die Dichtung über den Bund festgelegt ist, relativ zum Endoskop kippbar gelagert ist.

Die kippbewegliche Lagerung der Dichtungsaufnahme wird bei einer Ausgestaltungsform der Erfindung dadurch ermöglicht, daß die Dichtungsaufnahme als ein in eine Öffnung des Endoskops eingesetztes kugelförmiges Element oder kugelsegmentförmiges Element ausgebildet ist, das mit einer Öffnung zum Durchführen eines medizinischen Instruments versehenen ist. Bei dieser einfach und kostengünstig herstellbaren Lagerung wirkt die Dichtungsaufnahme quasi als Kugelgelenk.

Bei einer weiteren erfindungsgemäßen Ausführungsform ist die Dichtungsaufnahme kardanisch in einer Öffnung des Endoskops gelagert.

Um sicherzustellen, daß bei der kippbeweglichen Lagerung der Dichtungsaufnahme das Endoskop auch im Bereich der in der Öffnung des Endoskops gelagerten Dichtungsaufnahme vollständig abgedichtet ist, wird weiterhin vorgeschlagen, daß die Dichtungsaufnahme über ein zusätzliches Dichtelement, insbesondere einen O-Ring, gegenüber der Öffnung in der Endfläche des Endoskops abgedichtet ist. Eine zuverlässige Abdichtung der Öffnung in der Endfläche der Dichtung gegenüber dem medizinischen Instrument wird dadurch erzielt, daß der Durchmesser der Öffnung kleiner ist als der Durchmesser des aufzunehmenden medizinischen Instruments.

Die Kippbeweglichkeit des in der Öffnung der Endfläche angeordneten medizinischen Instruments kann dadurch verbessert werden, daß der die Öffnung in der Endfläche umgebende Rand der Endfläche im Querschnitt halbkreisförmig ausgebildet ist, so daß sich eine fast nur linienfömige abdichtende Anlage des Randes der Öffnung an dem medizinischen Instrument ergibt. Gegenüber einer einfachen zylindrischen Ausgestaltung dieses Randes ermöglicht die halbkreisförmige Ausgestaltung ein leichtes und widerstandsarmes Verschwenken des medizinischen Instruments.

Gemäß einer bevorzugten Ausführungsform der Erfindung findet das mit der Dichtung versehene Endoskop in der Proktoskopie, speziell in einem Rektoskop, beispielsweise einem Video-Rektoskop, Verwendung.

Gemäß einer praktischen Ausführungsform der erfindungsgemäßen Dichtungsanordnung ist das zusätzliche Dichtungselement eine Kreuzschlitz-Dichtung. Die Verwendung der an sich bekannten Kreuzschlitz-Dichtung gewährleistet eine sichere Abdichtung bei entferntem medizinischen Instrument, da in diesem Fall die durch den Kreuzschlitz voneinander getrennten Bereiche der Endfläche der Kreuzschlitz-Dichtung aufgrund der materialbedingten Rückstellkraft abdichtend aneinanderliegen. Statt der Kreuzschlitz-Dichtung sind auch andere Dichtungselemente, wie beispielsweise Ventile oder Rückschlagklappen verwendbar.

Gemäß einer bevorzugten Ausgestaltungsform der Erfindung wird vorgeschlagen, daß die Dichtung distalseitig an einer distalen Endfläche des Endoskops und das zusätzliche Dichtungselement proximalseitig an der distalen Endfläche des Endoskops festlegbar ist.

Das Festlegen des Bunds der Dichtung an der Außenseite des Dichtungsstutzens erfolgt gemäß einer Ausführungsform dadurch, daß der Bund der Dichtung mindestens eine Hinterschneidung des Dichtungsstutzens hintergreift.

Schließlich wird gemäß einer weiteren erfindungsgemäßen Ausführungsform vorgeschlagen, den Bund der Dichtung auf der Innenseite des Dichtungsstutzens festzulegen, wozu der Bund mindestens einen auf der Innenseite des Dichtungsstutzens angeordneten Vorsprung hintergreift.

Weitere Merkmale und Vorteile der Erfindung ergeben sich anhand der Beschreibung der zugehörigen Zeichnung, in der sechs Ausführungsbeispiele einer erfindungsgemäßen Dichtung nur beispielhaft schematisch dargestellt sind. In der Zeichnung zeigt:
- Fig. 1: ein Draufsicht auf eine erste Ausführungsform einer erfindungsgemäßen Dichtung;
- Fig. 2: einen Längsschnitt entlang der Schnittlinie II-II gemäß Fig. 1;
- Fig. 3: einen schematischen Längsschnitt durch eine erfindungsgemäße Dichtungsanordnung, eine zweite Ausführungsform einer erfindungsgemäßen Dichtung darstellend;
- Fig. 4a: eine Draufsicht auf eine Dichtungsanordnung gemäß Fig. 3 im zusammengebauten Zustand;
- Fig. 4b: eine Seitenansicht der Dichtungsanordnung gemäß Fig. 4a;
- Fig. 4c: eine Ansicht von unten der Dichtungsanordnung gemäß Fig. 4a und 4b;
- Fig. 5: einen Längsschnitt durch eine dritte Ausführungsform einer erfindungsgemäßen Dichtung;
- Fig. 6: eine vergrößerte Ansicht des Details VI gemäß Fig. 5;
- Fig. 7: einen schematischen Längsschnitt durch eine vierte Ausführungsform einer erfindungsgemäßen Dichtung;
- Fig. 8: eine schematische perspektivische und teilweise geschnittene Ansicht einer fünften Ausführungsform einer erfindungsgemäßen Dichtung und
- Fig. 9: eine Fig. 8 entsprechende Ansicht, eine sechste Ausführungsform darstellend.

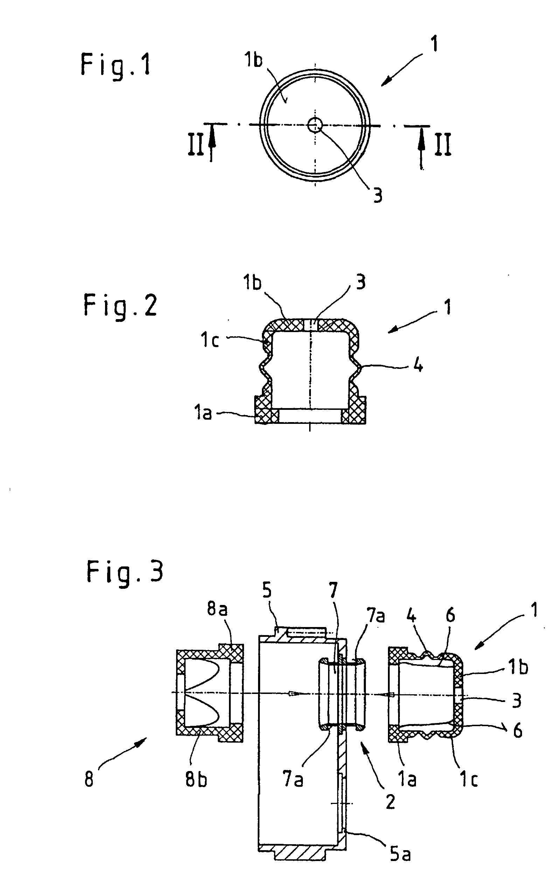

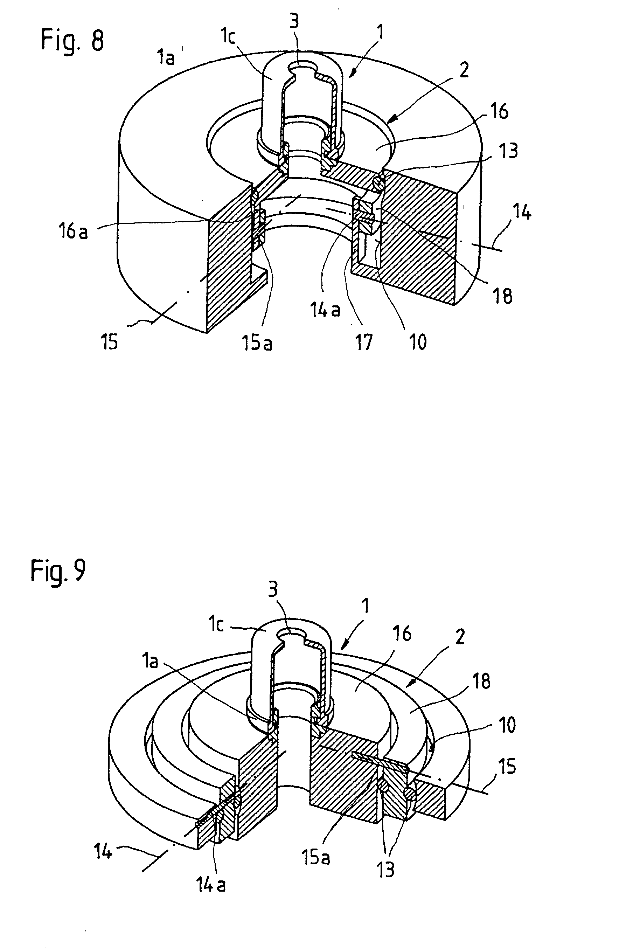

Die in den Abbildungen Fig. 1 und 9 dargestellten Dichtungen 1 bestehen aus einem Bund 1a zum Festlegen der Dichtung 1 an einer Dichtungsaufnahme 2 eines Endoskops, einer Endfläche 1b und einem den Bund 1a mit der Endfläche 1b vebindenden Dichtungsmantel 1c. Wie aus den Abbildungen weiterhin ersichtlich, ist in der Endfläche 1b eine Öffnung 3 ausgebildet. Bei der Verwendung der Dichtung 1 an einem Endoskop dient die Öffnung 3 zum Durchführen eines medizinischen Instruments.

Um eine wirksame Abdichtung zwischen dem einzuführenden medizinischen Instrument und der Öffnung 3 in der Endfläche 1b zu gewährleisten, ist der Durchmesser der Öffnung 3 so bemessen, daß er geringfügig kleiner ist als der Durchmesser des einzusetzenden medizinischen Instruments. Aufgrund der Materialelastizität der vorzugsweise aus einem Gummi-Material gefertigten Dichtung 1 schmiegt sich der Rand 3a der Öffnung 3 formschlüssig und abdichtend an das medizinische Instrument.

Bei den in den Abbildungen Fig. 1 bis 4c dargestellten Ausführungsformen ist der Dichtungsmantel 1c der Dichtung 1 als mit einer Falte 4 versehener Faltenbalg ausgebildet. Durch die Faltenbildung des Dichtungsmantels 1c wird der Dichtungsmantel 1c biegeweich, das heißt dehn- und stauchbar sowie kippbeweglich. Diese Biegeweichheit des Dichtungsmantels 1c ist vorteilhaft, da so das durch die Öffnung 3 der Endfläche 1b geführte medizinische Instrument während einer Untersuchung leicht in verschiedene radiale Richtungen verlagert werden kann, ohne daß das Material der Dichtung 1 diese Kippbewegungen beeinträchtigt.

Neben der Ausbildung des Faltenbalgs mit nur einer Falte 4 ist es selbstverständlich auch möglich, mehrere Falte 4 vorzusehen, die horizontal oder wendelförmig verlaufend im Material des Dichtungsmantels 1c ausgebildet sind. Die Biegeweichheit und somit auch die Kippbeweglichkeit erhöht sich mit zunehmender Faltenzahl. Zusätzlich zur Anzahl der Falten 4 läßt sich die Kippbeweglichkeit der Dichtung 1 auch durch die Höhe der einzelnen Falten 4 beeinflussen. Im einfachsten Fall weisen alle Falten 4 des Faltenbalgs im entspannten Zustand die gleiche Höhe auf. Jedoch besteht auch die Möglichkeit, zumindest eine Falte 4 mit einer im entspannten Zustand von der Höhe der anderen Falten 4 abweichenden Höhe auszubilden.

Die Abbildung Fig. 3 zeigt eine zweite Ausführungsform der Dichtung 1, deren Dichtungsmantel 1c als Faltenbalg ausgebildet ist, in einer Dichtungsanordnung zur Anordnung an einer distalseitigen Abschlußkappe 5 eines Endoskops. Von der in der Abbildung Fig. 2 dargestellten ersten Ausführungsform der Dichtung 1 unterscheidet sich die Ausführungsform gemäß Fig. 3 dadurch, daß der Bund 1a und die Endfläche 1b der Dichtung 1 über mindestens ein nicht dehnbares Begrenzungselement 6, vorzugsweise ein Seil, miteinander verbunden sind.

Das Begrenzungselement 6 dient dazu, ein Überdehnen des Dichtungsmantels 1c zu verhindern, was zu einer Zerstörung und somit Undichtigkeit des Dichtungsmantels 1c führen kann. Diese Begrenzung der axialen Dehnung des Faltenbalgs ist insbesondere beim Herausziehen des medizinischen Instruments aus der Öffnung 3 der Endfläche 1b und bei extremen Kippbewegungen des medizinischen Instruments erforderlich. Insbesondere zur Begrenzung der Längsdehnung bei extremen Kippbewegungen kann es vorteihaft sein, wenn mehrere Begrenzungselemente 6, beispielsweise drei, um die Öffnung 3 herum verteilt vorgesehen sind.

Zum Festlegen der Dichtung 1 an der Abschlußkappe 5 ist bei dem dargestellten Ausführungsbeispiel die Dichtungsaufnahme 2 der Abschlußkappe 5 als Dichtungsstutzen 7 ausgebildet. Im montierten Zustand hintergreift der Bund 1a der Dichtung 1 eine Hinterschneidung 7a des Dichtungsstutzens 7 und fixiert so die Dichtung 1 am Endoskop. Um sicherzustellen, daß das Endoskop auch dann abgedichtet ist, wenn kein medizinisches Instrument in die Dichtung 1 eingesetzt ist, weist die Dichtungsanordnung ein zusätzliches Dichtungselement 8 auf, das von der proximalen Seite her auf den Dichtungsstutzen 7 der distalen Endfläche 5a aufgesetzt ist. Auch dieses zusätzliche Dichtungselement 8, das im dargestellten Ausführungsbeispiel als Kreuzschlitz-Dichtung 8b ausgebildet ist, wird über einen Bund 8a an einer Hinterschneidung 7a des Dichtungsstutzen 7 festgelegt.

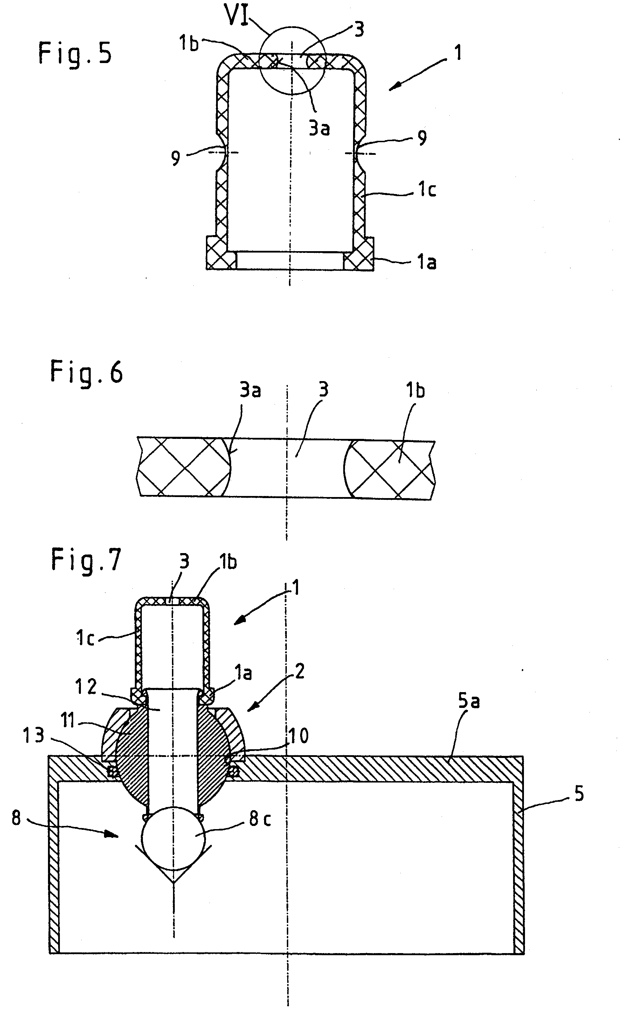

Neben dieser dargestellten Festlegung des Bunds 1a der Dichtung 1 auf der Außenseite des Dichtungsstutzens 7 ist es selbstverständlich auch möglich, den Bund 1a auf der Innenseite des Dichtungsstutzens 7 festzulegen. In diesem Fall ist auf der Innenseite des Dichtungsstutzens 7 mindestens ein nach innen ragender Vorsprung angeordnet, den der Bund 1a der Dichtung 1 fixierend hintergreift. Ebenso besteht die Möglichkeit, das zusätzliche Dichtungselement 8 beispielsweise als Rückschlagklappe oder als Ventil 8c auszubilden, wie dies in Fig. 7 dargestellt ist.

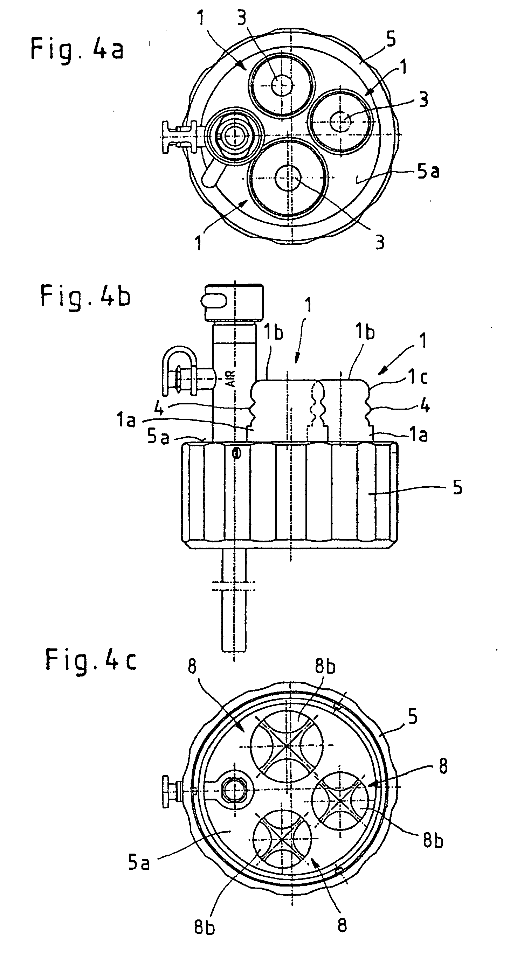

Die Abbildungen Fig. 4a bis 4c zeigen eine prinzipiell Fig. 3 entsprechende Dichtungsanordnung. Diesen Darstellungen ist zu entnehmen, daß mehrere Faltenbalg-Dichtungen 1 an der Abschlußkappe 5 festlegbar sind.

Bei der in Abbildung Fig. 5 dargestellten dritten Ausführungsform der Dichtung 1 wird die Kippbeweglichkeit der Endfläche 1b der Dichtung 1 relativ zum Endoskop dadurch erzielt, daß im Material des Dichtungsmantels 1c an einer vorgegebenen Stelle mindestens ein eine Soll-Knickstelle bildender Bereich mit einer verringerten Wandstärke 9 ausgebildet ist. Ähnlich wie bei der Ausbildung der Falten 4 des Faltenbalgs läßt sich die Biegeweichheit der Dichtung 1 durch die Anzahl und/oder Anordnung der Bereiche mit einer verringerten Wandstärke 9 zueinander variieren. Bei der in Fig. 5 dargestellten Ausführungsform ist der Bereich mit einer verringerten Wandstärke 9 als horizontal umlaufender Bereich im Material des Dichtungsmantels 1c ausgebildet.

Gemäß alternativen Ausgestaltungsformen können diese Bereiche mit einer verringerten Wandstärke 9 auch als einzelne voneinander beabstandete und/oder zueinander versetzt angeordnete Teilbereiche oder als wendelförmig umlaufender Bereich im Material des Dichtungsmantels 1c ausgebildet sein.

Das in Fig. 6 vergrößert dargestellte Detail der Abbildung Fig. 5 zeigt, wie der die Öffnung 3 zur Aufnahme des medizinischen Instruments umgebender Rand 3a der Endfläche 1b ausgebildet sein kann, um das medizinische Instrument besonders kippbeweglich zu lagern. Der bei dieser Ausführungsform im Querschnitt halbkreisförmig ausgebildete Rand 3a bewirkt eine quasi linienförmige formschlüssige Anlage an dem in die Öffnung 3 eingesetzten medizinischen Instrument. Aufgrund dieser nur linienförmigen Berührung ist das Instrument besonders kippbeweglich in der Öffnung 3 gelagert, da beim Verschwenken des Instruments nahezu keine Materialwiderstände des Dichtungsmaterials zu überwinden sind. Die dargestellte Ausbildung des Randes 3a ist selbstverständlich bei allen in den Abbildungen Fig. 1 bis Fig. 8 dargestellten Ausführungsformen verwendbar.

In den Abbildungen Fig. 7 bis 9 sind drei weitere Ausführungsformen dargestellt, wie die Endfläche 1b der Dichtung 1 relativ zum Endoskop kippbeweglich lagerbar ist. Bei diesen Ausgestaltungen der Dichtung 1 erfolgt das Kippen indirekt, und zwar dadurch, daß die Dichtungsaufnahme 2, an der der Bund 1a der Dichtung 1 festgelegt ist, relativ zum Endoskop kippbar gelagert ist.

Gemäß der in Fig. 7 dargestellten vierten Ausführungsform wird die kippbewegliche Lagerung der Dichtungsaufnahme 2 dadurch erzielt, daß die Dichtungsaufnahme 2 als in eine Öffnung 10 des Endoskops einsetzbares kugelförmiges Element 11 ausgebildet ist, das in der Art eines Kugelgelenks ein Verschwenken des in die Dichtung 1 eingesetzten medizinischen Instruments ermöglicht. Bei der dargestellten Ausführungsform wird der Bund 1a der Dichtung 1 nicht direkt an dem kugelförmigen Element 11 festgelegt, sondern an einem in das kugelförmige Element 11 eingesetzten und zusammen mit diesem die Dichtungsaufnahme 2 bildenden Stutzen 12. Selbstverständlich ist es aber auch möglich, den Bund 1a der Dichtung 1 direkt an dem kugelförmigen Element 11 festzulegen.

Eine weitere, mit der Ausgestaltung gemäß Fig. 7 vergleichbare kippbewegliche Lagerung der Dichtungsaufnahme 2 ist auch dadurch erzielbar, daß die Dichtungsaufnahme 2 nicht als vollständig kugelförmiges Element 11, sondern nur als kugelsegmentförmiges Element ausgestaltet wird, wobei das Kugelsegment vorzugsweise ein den Mittelbereich einer Kugel umfassendes Segment ist, um ein Herausfallen aus der Öffnung in der Endfläche des Endoskops zu verhindern.

Um sicherzustellen, daß auch bei der Verwendung der kippbar in der Öffnung 10 des Endoskops gelagerten Dichtungsaufnahme 2 das Endoskop vollständig und zuverlässig abgedichtet ist, ist bei der Ausgestaltungsform gemäß Fig. 7 das kugelförmige Element 11 über eine beispielsweise als O-Ring 13 ausgebildete Dichtung gegenüber dem Rand der Öffnung 10 abgedichtet.

Die Abbildungen Fig. 8 und 9 zeigen schließlich fünfte und sechste Ausführungsformen der Dichtung 1, bei denen ähnlich wie bei der Ausführungsform gemäß Fig. 7 die Dichtungsaufnahme 2 kippbeweglich in der Öffnung 10 des Endoskops gelagert ist, um das Verschwenken der Endfläche 1b der Dichtung 1 relativ zum Endoskop zu ermöglichen. Bei den Ausgestaltungen gemäß Fig.8 und 9 ist die Dichtungsaufnahme 2 jeweils kardanisch in der Öffnung 10 des Endoskops gelagert.

Wie aus den Prinzipskizzen gemäß Fig. 8 und 9 ersichtlich, ist die Dichtungsaufnahme 2 als um zwei Schwenkachsen 14 und 15 kippbare Scheibe 16 ausgebildet, die in der Öffnung 10 des Endoskops angeordnet ist.

Zur Ausbildung der kardanischen Lagerung sind bei der in Fig 8 dargestellten ersten Ausführungsform einer kardanischen Lagerung der Dichtungsaufnahme 2 am Rand der Öffnung 10 des Endoskops zwei Tragarme 17 angeordnet, die mittels Achsstummeln 14a einen um die erste Schwenkachse 14 kippbaren Schwenkrahmen 18 tragen. Über zwei Befestigungslaschen 16a und Achsstummel 15a ist die kippbare Scheibe 16 um die zweite Schwenkachse 15 kippbar an dem Schwenkrahmen 18 lagerbar, wobei die beiden Schwenkachsen 14 und 15 in einem Winkel von 90° zueinander angeordnet sind.

Die Abdichtung der kippbaren Scheibe 16 gegenüber der Öffnung 10 des Endoskops erfolgt bei dieser Ausführungsform über einen O-Ring 13, wobei der o-Ring 13 an der kippbaren Scheibe 16 gelagert ist und die Dichtungsfläche der Öffnung 10 vorzugsweise konkav gewölbt ausgebildet ist, um das Verschwenken der kippbaren Scheibe 16 zu erleichtern. Selbstverständlich ist es auch möglich, den O-Ring 13 in der Öffnung 10 zu lagern und die die Dichtfläche bildende radiale Außenfläche der kippbaren Scheibe 16 konkav gewölbt auszubilden.

Bei der in Fig. 9 dargestellten zweiten Ausführungsform einer kardanisch gelagerten Dichtungsaufnahme 2 sind die kippbare Scheibe 16 und der Schwenkrahmen 18 konzentrisch zueinander in der Öffnung 10 des Endoskop angeordnet. Wie aus der schematischen Darstellung ersichtlich, ist bei dieser Ausgestaltungsform der Schwenkrahmen 18 direkt über die Achsstummel 14a verschwenkbar in der Öffnung 10 in der Endfläche des Endoskops gelagert. Die kippbare Scheibe 16 ist ihrerseits innerhalb des Schwenkrahmens 18 angeordnet und über die Achsstummel 15a verschwenkbar im Schwenkrahmen 18 gelagert. Auch bei dieser Ausführungsform sind die Schwenkachsen 14 und 15 in einem Winkel von 90° zueinander angeordnet.

Um auch bei dieser kardanisch in der Öffnung 10 des Endoskops gelagerten Dichtungsaufnahme 2 sicherzustellen, daß das Endoskop vollständig und zuverlässig abgedichtet ist, sind bei der Ausgestaltungsform gemäß Fig. 9 einerseits die kippbare Scheibe 16 gegenüber dem Schwenkrahmen 18 und andererseits der Schwenkrahmen 18 gegenüber dem Rand der Öffnung 10 des Endoskops über beispielsweise als O-Ringe 13 ausgebildete Dichtungen abgedichtet. Wie aus der Abbildung ersichtlich, sind die O-Ringe 13 am Schwenkrahmen 18 gelagert und die Dichtflächen am Rand der Öffnung 10 des Endoskops einerseits und an der kippbaren Scheibe 16 andererseits, gegen die die O-Ringe 13 abdichtend anliegen, in den zugehörigen achsnahen Bereichen plan ausgeführt, wohingegen sie in den von den Achsstummeln 14a und 15a ausgehenden entfernteren Bereichen zunehmend konkav gewölbt ausgebildet sind, um die einen Kreisabschnitt beschreibende Bewegung des O-Rings 13 an der kippbaren Scheibe 16 bzw. am Schwenkrahmen 18 aufnehmen zu können.

Alternativ kann die abdichtung zwischen der Öffnung 10 und dem Schwenkrahmen 18 auch so ausgebildet sein, daß die O-Ringe 13 einerseits in der Öffnung 10 und andererseits an der kippbaren Scheibe 16 gelagert sind. Die entsprechenden Dichtflächen am Schwenkrahmen 18 sind dann so ausgebildet, daß sie in den zugehörigen achsnahen Bereichen plan ausgebildet sind und in den von den Achsstummeln 14a und 15a ausgehenden entfernten Bereichen zunehmend konkav gewölbt ausgebildet sind.

Die solchermaßen gemäß den Abbildungen Fig. 1 bis 9 ausgebildeten Dichtungen 1 zeichnen sich dadurch aus, daß sie einerseits dem durch die Öffnung 3 der Endfläche 1b hindurchgeführten medizinischen Instrument einen ausreichenden Bewegungsspielraum einräumen und andererseits eine zuverlässige Abdichtung gewährleisten.

### Bezugszeichen liste

- 1: Dichtung
- 1a: Bund
- 1b: Endfläche
- 1c: Dichtungsmantel
- 2: Dichtungsaufnahme
- 3: Öffnung
- 3a: Rand
- 4: Falte
- 5: Abschlußkappe
- 5a: distale Endfläche
- 6: Begrenzungselement
- 7: Dichtungsstutzen
- 7a: Hinterschneidung
- 8: Dichtungselement
- 8a: Bund
- 8b: Kreuzschlitz-Dichtung
- 8c: Ventil
- 9: Bereich verringerter Wandstärke
- 10: Öffnung
- 11: kugelförmiges Element
- 12: Stutzen
- 13: O-Ring
- 14: Schwenkachse
- 14a: Achsstummel
- 15: Schwenkachse
- 15a: Achsstummel
- 16: kippbare Scheibe
- 16a: Befestigungslasche
- 17: Tragarm
- 18: Schwenkrahmen

## Patentansprüche

1. Dichtungsanordnung für ein Endoskop, insbesondere ein Rektoskop, umfassend eine Dichtungsaufnahme (2) des Endoskops, eine Dichtung (1) und ein weiteres Dichtungselement (8), wobei die Dichtung (1) einen Bund (1a) zum Festlegen an der Dichtungsaufnahme (2) des Endoskops, eine mit einer Öffnung (3) zum Durchführen eines medizinischen Instruments versehene Endfläche (1b) sowie einen den Bund trat und die Endfläche (1b) miteinander verbindenden Dichtungsmantel (1c) aufweist und die Endfläche (1b) relativ zur Dichtungsaufnahme (2) des Endoskops kippbar gelagert ist,
**dadurch gekennzeichnet,**
**dass** die Streckung des Dichtungsmantels (1c) in axialer Richtung begrenzbar ist.

2. Dichtungsanordnung nach Anspruch 1, **dadurch gekennzeichnet, daß** der Bund (1a) und die Endfläche (1b) über mindestens ein nicht dehnbares Begrenzungselement (6) miteinander verbunden sind.

3. Dichtungsanordnung nach Anspruch 2, **dadurch gekennzeichnet, daß** die Begrenzungselemente (6) gleichmäßig um die Öffnung (3) zum Durchführen eines medizinischen Instruments angeordnet sind.

4. Dichtungsanordnung nach Anspruch 2 der 3 **dadurch gekennzeichnet, daß** das Begrenzungselement (6) als Seil ausgebildet ist.

5. Dichtungsanordnung nach mindestens einem der Ansprüche 1 bis 4, **dadurch gekennzeichnet, dass** die Dichtungsaufnahme (2) als Dichtungsstutzen (7) ausgebildet ist.

6. Dichtungsanordnung nach mindestens einem der Ansprüche 1 bis 5, **dadurch gekennzeichnet, daß** der Bund (1a) der Dichtung (1) mindestens eine Hinterschneidung (7a) des Dichtungsstutzens (7) hintergreifend an der Außenseite des Dichtungsstutzens (7) festlegbar ist.

7. Dichtungsanordnung nach mindestens einem der Ansprüche 1 bis 5, **dadurch gekennzeichnet, daß** der Bund (1a) der Dichtung (1) auf der Innenseite des Dichtungsstutzens (7) festlegbar ist, wobei der Bund (1a) mindestens einen auf der Innenseite des Dichtungsstutzens (7) angeordneten Vorsprung hintergreift.

8. Dichtungsanordnung nach mindestens einem der Ansprüche 1 bis 7, **dadurch gekennzeichnet, daß** der Dichtungsmantel (1c) als mit mindestens einer Falte (4) versehener Faltenbalg ausgebildet ist.

9. Dichtungsanordnung nach Anspruch 8, **dadurch gekennzeichnet, daß** die mindestens eine Falte (4) wendelfömig umlaufend im Material des Dichtungsmantels (1c) ausgebildet ist.

10. Dichtungsanordnung nach Anspruch 8 oder 9, **dadurch gekennzeichnet, daß** der Faltenbalg mehrere Falten (4) aufweist.

11. Dichtungsanordnung nach Anspruch 10 **dadurch gekennzeichnet, daß** alle Falten (4) im entspannten Zustand die gleiche Höhe aufweisen.

12. Dichtungsanordnung nach Anspruch 10, **dadurch gekennzeichnet, daß** zumindest einzelne Falten (4) im entspannten Zustand eine von den anderen Falten (4) abweichende Höhe aufweisen.

13. Dichtungsanordnung nach mindestens einem der Ansprüche 1 bis 7, **dadurch gekennzeichnet, daß** der Dichtungsmantel (1c) mindestens eine Soll-Knickstelle bildend an zumindest einer vorgegebenen Stelle einen Bereich mit einer verringerten Wandstärke (9) aufweist.

14. Dichtungsanordnung nach Anspruch 13, **dadurch gekennzeichnet, daß** im Material des Dichtungsmantels (1c) mehrere Bereiche mit verringerter Wandstärke (9) ausgebildet sind, wobei diese Bereiche mit verringerter Wandstärke (9) in der Höhe und/oder über den Umfang des Dichtungsmantels (1c) versetzt zueinander angeordnet sind.

15. Dichtungsanordnung nach Anspruch 13 oder 14, **dadurch gekennzeichnet, daß** der mindestens eine Bereich mit verringerter Wandstärke (9) als horizontal umlaufender Bereich im Material des Dichtungsmantels (1c) ausgebildet ist.

16. Dichtungsanordnung nach Anspruch 13 oder 14, **dadurch gekennzeichnet, daß** der mindestens eine Bereich mit verringerter Wandstärke (9) als wendeiförmig umlaufender Bereich im Material des Dichtungsmantels (1c) ausgebildet ist.

17. Dichtungsanordnung nach Anspruch 1, **dadurch gekennzeichnet, daß** die Dichtungsaufnahme (2) kippbar am Endoskop gelagert ist.

18. Dichtungsanordnung nach Anspruch 17, **dadurch gekennzeichnet, daß** die Dichtungsaufnahme (2) als ein in eine Öffnung (10) des Endoskops eingesetztes kugelförmiges Element (11) oder kugelsegmentförmiges Element ausgebildet ist, das mit einer Öffnung zum Durchführen eines medizinischen Instruments versehen ist.

19. Dichtungsanordnung nach Anspruch 17, **dadurch gekennzeichnet, daß** die Dichtungsaufnahme (2) kardanisch in der Öffnung (10) des Endoskops gelagert ist.

20. Dichtungsanordnung nach mindestens einem der Ansprüche 17 bis 19, **dadurch gekennzeichnet, daß** die Dichtungsaufnahme (2) über ein zusätzliches Dichtelement, insbesondere einen O-Ring (13), gegenüber der Öffnung (10) des Endoskops abgedichtet ist.

21. Dichtungsanordnung nach mindestens einem der Ansprüche 1 bis 20, **dadurch gekennzeichnet, daß** der Durchmesser der Öffnung (3) in der Endfläche (1b) kleiner ist als der Durchmesser des jeweils aufzunehmenden medizinischen Instruments.

22. Dichtungsanordnung nach mindestens einem der Ansprüche 1 bis 21, **dadurch gekennzeichnet, daß** ein die Öffnung (3) in der Endfläche (1b) umgebender Rand (3a) der Endfläche (1b) im Querschnitt halbkreisförmig ausgebildet ist.

23. Dichtungsanordnung nach mindestens einem der Ansprüche 1 bis 22, **dadurch gekennzeichnet, daß** das zusätzliche Dichtungselement (8) eine Kreuzschlitz-Dichtung (8b) ist.

24. Dichtungsanordnung nach mindestens einem der Ansprüche 1 bis 23, **dadurch gekennzeichnet, daß** das zusätzliche Dichtungselement (8) als Ventil (8c) oder Rückschlagklappe ausgebildet ist.

25. Dichtungsanordnung nach mindestens einem der Ansprüche 1 bis 24, **dadurch gekennzeichnet, daß** die Dichtung (1) distalseitig an einer distalen Endfläche (5a) des Endoskops und das zusätzliche Dichtungselement (8) proximalseitig an der distalen Endfläche (5a) des Endoskops festlegbar ist.

## Claims

1. Sealing arrangement for an endoscope, in particular for a rectoscope, comprising a seal-receiving seat (2) of the endoscope, a seal (1), and a further sealing element (8), which seal (1) has a flange (1a) for securing on the seal-receiving seat (2) of the endoscope, an end face (1b) provided with an opening (3) for the passage of a medical instrument, and a seal jacket (1c) connecting the flange (1a) and the end face (1b) to one another, and the end face (1b) is mounted so that it is tiltable relative to the seal-receiving seat (2) of the endoscope, **characterized in that** the stretching of the seal jacket (1c) in the axial direction can be limited.

2. Sealing arrangement according to Claim 1, **characterized in that** the flange (1a) and the end face (1b) are connected to one another via at least one non-extendable limiting element (6).

3. Sealing arrangement according to Claim 2, **characterized in that** the limiting elements (6) are arranged uniformly about the opening (3) for the passage of a medical instrument.

4. Sealing arrangement according to Claim 2 or 3, **characterized in that** the limiting element (6) is designed as a cord.

5. Sealing arrangement according to at least one of Claims 1 to 4, **characterized in that** the seal-receiving seat (2) is designed as a seal connection piece (7).

6. Sealing arrangement according to at least one of Claims 1 to 5, **characterized in that** the flange (1a) of the seal (1) can be fixed on the outside of the seal connection piece (7), engaging behind at least one undercut (7a) of the seal connection piece (7).

7. Sealing arrangement according to at least one of Claims 1 to 5, **characterized in that** the flange (1a) of the seal (1) can be fixed on the inside of the seal connection piece (7), said flange (1a) engaging behind at least one projection arranged on the inside of the seal connection piece (7).

8. Sealing arrangement according to at least one of Claims 1 to 7, **characterized in that** the seal jacket (1c) is designed as a bellows provided with at least one fold (4).

9. Sealing arrangement according to Claim 8, **characterized in that** the at least one fold (4) is designed extending helically in the material of the seal jacket (1c).

10. Sealing arrangement according to Claim 8 or 9, **characterized in that** the bellows has a plurality of folds (4).

11. Sealing arrangement according to Claim 10, **characterized in that** all the folds (4) have the same height in the relaxed state.

12. Sealing arrangement according to Claim 10, **characterized in that** at least individual folds (4), in the relaxed state, have a height differing from that of the other folds (4).

13. Sealing arrangement according to at least one of Claims 1 to 7, **characterized in that** the seal jacket (1c) has at least one intended kink point forming, at at least one predetermined point, an area with a reduced wall thickness (9).

14. Sealing arrangement according to Claim 13, **characterized in that** a plurality of areas with reduced wall thickness (9) are formed in the material of the seal jacket (1c), these areas with reduced wall thickness (9) being offset in relation to one another in height and/or about the circumference of the seal jacket (1c).

15. Sealing arrangement according to Claim 13 or 14, **characterized in that** the at least one area with reduced wall thickness (9) is designed as an area extending horizontally in the material of the seal jacket (1c).

16. Sealing arrangement according to Claim 13 or 14, **characterized in that** the at least one area with reduced wall thickness (9) is designed as an area extending helically in the material of the seal jacket (1c).

17. Sealing arrangement according to Claim 1, **characterized in that** the seal-receiving seat (2) is mounted tiltably on the endoscope.

18. Sealing arrangement according to Claim 17, **characterized in that** the seal-receiving seat (2) is designed as a ball-shaped element (11), or as an element in the shape of a segment of a sphere, which element (11) is fitted into an opening (10) of the endoscope and is provided with an opening for the passage of a medical instrument.

19. Sealing arrangement according to Claim 17, **characterized in that** the seal-receiving seat (2) is mounted as a cardan joint in the opening (10) of the endoscope.

20. Sealing arrangement according to at least one of Claims 17 to 19, **characterized in that** the seal-receiving seat (2) is sealed off in relation to the opening (10) of the endoscope via an additional sealing element, in particular via an O-ring seal (13).

21. Sealing arrangement according to at least one of Claims 1 to 20, **characterized in that** the diameter of the opening (3) in the end face (1b) is smaller than the diameter of the respective medical instrument to be received.

22. Sealing arrangement according to at least one of Claims 1 to 21, **characterized in that** an edge (3a) of the end face (1b) surrounding the opening (3) in the end face (1b) is designed in cross section as a semicircle.

23. Sealing arrangement according to at least one of Claims 1 to 22, **characterized in that** the additional sealing element (8) is a cross-slotted seal (8b).

24. Sealing arrangement according to at least one of Claims 1 to 23, **characterized in that** the additional sealing element (8) is designed as a valve (8c) or nonreturn valve.

25. Sealing arrangement according to at least one of Claims 1 to 24, **characterized in that** the seal (1) on the distal side can be fixed on a distal end face (5a) of the endoscope and the additional sealing element (8) on the proximal side can be fixed on the distal end face (5a) of the endoscope.

## Revendications

1. Système d'étanchéité pour un endoscope, en particulier un rectoscope, comprenant un logement pour garniture d'étanchéité (2) de l'endoscope, une garniture d'étanchéité (1) et un autre élément d'étanchéité (8), dans lequel la garniture d'étanchéité (1) comprend une collerette (1a) à fixer contre le logement pour garniture d'étanchéité (2) de l'endoscope, une surface d'extrémité (1b) munie d'un orifice (3) destiné au passage d'un instrument médical, ainsi qu'une gaine d'étanchéité (1c) reliant l'une à l'autre la collerette (1a) et la surface d'extrémité (1b), et la surface d'extrémité (1b) est logée de manière basculante par rapport au logement pour garniture d'étanchéité (2) de l'endoscope, **caractérisé en ce que** l'allongement de la gaine d'étanchéité (1c) peut être limité dans la direction axiale.

2. Système d'étanchéité selon la revendication 1, **caractérisé en ce que** la collerette (1a) et la surface d'extrémité 1b) sont reliées l'une à l'autre par au moins un élément de limitation (6) non extensible.

3. Système d'étanchéité selon la revendication 2, **caractérisé en ce que** les éléments de limitation (6) sont disposés régulièrement autour de l'orifice (3) destiné au passage de l'instrument médical.

4. Système d'étanchéité selon la revendication 2 ou 3, **caractérisé en ce que** l'élément de limitation (6) est conçu sous forme de câble.

5. Système d'étanchéité selon l'une quelconque des revendications 1 à 4, **caractérisé en ce que** le logement pour garniture d'étanchéité (2) est conçu sous forme de manchon d'étanchéité (7).

6. Système d'étanchéité selon l'une quelconque des revendications 1 à 5, **caractérisé en ce que** la collerette (1a) de la garniture d'étanchéité (1) peut être fixée contre la face extérieure du manchon d'étanchéité (7) en s'engageant derrière au moins une contre-dépouille (7a) du manchon d'étanchéité (7).

7. Système d'étanchéité selon l'une quelconque des revendications 1 à 5, **caractérisé en ce que** la collerette (1a) de la garniture d'étanchéité (1) est fixée sur la face intérieure du manchon d'étanchéité (7), moyennant quoi la collerette (1a) s'engage derrière au moins une saillie agencée sur la face intérieure du manchon d'étanchéité (7).

8. Système d'étanchéité selon l'une quelconque des revendications 1 à 7, **caractérisé en ce que** la gaine d'étanchéité (1c) est conçue sous forme de tuyau annelé muni d'au moins un pli (4).

9. Système d'étanchéité selon la revendication 8, **caractérisé en ce que** le au moins un pli (4) est réalisé dans la matière de la gaine d'étanchéité (1c) sous forme de spirale périphérique.

10. Système d'étanchéité selon la revendication 8 ou 9, **caractérisé en ce que** le tuyau annelé comprend plusieurs plis (4).

11. Système d'étanchéité selon la revendication 10, **caractérisé en ce que** tous les plis (4) d'un tuyau annelé ont la même hauteur à l'état détendu.

12. Système d'étanchéité selon la revendication 10, **caractérisé en ce que**, au moins quelques plis (4) du tuyau annelé ont une hauteur différente des autres plis (4) à l'état détendu.

13. Système d'étanchéité selon l'une quelconque des revendications 1 à 7, **caractérisé en ce que** la gaine d'étanchéité (1c) comprend au moins une zone avec une épaisseur de paroi réduite (9) formant une zone de flexion théorique au niveau d'au moins un emplacement prédéfini.

14. Système d'étanchéité selon la revendication 13, **caractérisé en ce que** plusieurs zones à épaisseur de paroi réduite (9) sont réalisées dans la matière de la gaine d'étanchéité (1c), ces zones à épaisseur de paroi réduite (9) étant décalées les unes par rapport aux autres sur la hauteur et/ou sur le pourtour de la gaine d'étanchéité (1c)

15. Système d'étanchéité selon la revendication 13 ou 14, **caractérisé en ce que**, au moins une zone à épaisseur de paroi réduite (9) est réalisée dans la matière de la gaine d'étanchéité (1c) sous forme de zone horizontalement périphérique.

16. Système d'étanchéité selon la revendication 13 ou 14, **caractérisé en ce que**, au moins une zone à épaisseur de paroi réduite (9) est réalisée dans la matière de la gaine d'étanchéité (1c) sous forme de zone périphérique en spirale.

17. Système d'étanchéité selon la revendication 1, **caractérisé en ce que** le logement pour garniture d'étanchéité (2) est posé de manière basculante au niveau de l'endoscope.

18. Système d'étanchéité selon la revendication 17, **caractérisé en ce que** le logement pour garniture d'étanchéité (2) est conçu sous forme d'élément sphérique (11) ou d'élément en forme de segment sphérique, inséré dans un orifice (10) de l'endoscope, lequel élément est muni d'un orifice pour le passage d'un instrument médical.

19. Système d'étanchéité selon la revendication 17, **caractérisé en ce que** le logement pour garniture d'étanchéité (2) est posé à la manière d'un joint de cardan dans l'orifice (10) de l'endoscope.

20. Système d'étanchéité selon l'une quelconque des revendications 17 à 19, **caractérisé en ce que** le logement pour garniture d'étanchéité (2) est rendu étanche par rapport à l'orifice (10) de l'endoscope par un élément d'étanchéité supplémentaire, en particulier un joint torique (13).

21. Système d'étanchéité selon l'une quelconque des revendications 1 à 20, **caractérisé en ce que** le diamètre de l'orifice (3) dans la surface d'extrémité (1b) est inférieur au diamètre de l'instrument médical à recevoir dans chaque cas.

22. Système d'étanchéité selon l'une quelconque des revendications 1 à 21, **caractérisé en ce qu'**un bord (3a) de la surface d'extrémité (1b) entourant l'orifice (3) dans la surface d'extrémité (1b) est réalisé avec une section de forme semi-circulaire.

23. Système d'étanchéité selon l'une quelconque des revendications 1 à 22, **caractérisé en ce que** l'élément d'étanchéité (8) supplémentaire est un joint d'étanchéité à fente cruciforme (8b).

24. Système d'étanchéité selon l'une quelconque des revendications 1 à 23, **caractérisé en ce que** l'élément d'étanchéité (8) supplémentaire est conçu sous forme de soupape (8c) ou de clapet de retenue.

25. Système d'étanchéité selon l'une quelconque des revendications 1 à 24, **caractérisé en ce que** la garniture d'étanchéité (1), du côté distal, peut être fixée au niveau d'une surface d'extrémité distale (5a) de l'endoscope et l'élément d'étanchéité (8) supplémentaire, du côté proximal, peut être fixé au niveau de la surface d'extrémité distale (5a) de l'endoscope.
